# EUROPÄISCHE PATENTANMELDUNG

(11) **EP 2 123 229 A1**
(43) Veröffentlichungstag der Anmeldung: **25.11.2009**
(21) Anmeldenummer: 08156468.4
(22) Anmeldetag: 19.05.2008
(51) Int. Cl.: A61B 17/15, A61B 19/00

(54) **Verbindung von Schneidblöcken**

(71) Anmelder: BrainLAB AG, 85622 Feldkirchen (DE)
(72) Erfinder: Warkentine, Blaine, Draper, 84020 (US); Plassky, Norman, 99099, Erfurt (DE); Millahn, Manuel, 80799 München (DE)
(74) Vertreter: Schwabe - Sandmair - Marx

(57) **Zusammenfassung**

Die vorliegende Anmeldung betrifft eine chirurgische Verbindungsvorrichtung zum Verbinden eines ersten und zweiten Schneidblocks, die jeweils der Durchführung eines Schnitts an einer ersten und zweiten anatomischen Struktur dienen, mit einem ersten Anbringabschnitt zum Anbringen der chirurgischen Verbindungsvorrichtung an dem ersten Schneidblock durch mechanische Verbindung und mit einem zweiten Anbringabschnitt zum Anbringen an dem zweiten Schneidblock (200) durch eine lösbare mechanische Verbindung, mit einem Beabstandungsabschnitt, der den ersten Anbringabschnitt von dem zweiten Anbringabschnitt beabstandet.

## Beschreibung

Die vorliegende Anmeldung betrifft die Verbindung von Schneidblöcken. Schneidblöcke werden in der Chirurgie, insbesondere zum Schneiden von Körperstrukturen (Knochen, insbesondere Femur und Tibia) eingesetzt.

Vorzugsweise wird die Erfindung bei der bildgestützten Navigation (IGS bzw. "image guided surgery") eingesetzt. Das Anbringen von Markervorrichtungen an einer Körperstruktur, z.B. eines Referenzsternes an einem Knochen und auch das Anbringen einer Markervorrichtung an einem Schneidblock erlauben das Navigieren eines Schneidblocks relativ zur Körperstruktur und somit das exakte Platzieren des Schneidblocks relativ zur Körperstruktur an einer geplanten Stelle. Um den Schneidblock exakt zu platzieren, wird ein Einstellmechanismus eingesetzt, der ebenfalls am Knochen fixiert wird. Gemäß dem Stand der Technik wird für jeden Schneidblock ein Einstellmechanismus an der Körperstruktur fixiert.

Aufgabe der Erfindung ist es bei dem Einsatz von zwei oder mehr Schneidblöcken zu ermöglichen, dass die Anzahl der Fixiervorgänge zum Fixieren der Einstellmechanismen an der Körperstruktur und/oder die Anzahl der eingesetzten Einstellmechanismen verringert wird.

Vorteilhaft wird erfindungsgemäß die Körperstruktur mehr geschont als im Stand der Technik, da weniger Fixierungen stattfinden. Vorteilhaft ist der Raumbedarf geringer, da weniger Einstellmechanismen, insbesondere nur ein Einstellmechanismus benötigt wird. Vorteilhaft kann durch die Erfindung die Operationszeit verkürzt werden und somit die Belastung für den Patienten verringert werden.

Vorstehend genannte Aufgabe wird durch die Gegenstände der unabhängigen Ansprüche gelöst. Vorteilhafte Weiterbildungen gehen aus den abhängigen Ansprüchen hervor.

Vorzugsweise betrifft die Erfindung eine chirurgische Verbindungsvorrichtung zum Verbinden eines ersten und eines zweiten Schneidblocks. Vorzugsweise wird die chirurgische Verbindungsvorrichtung im Bereich eines Gelenks eingesetzt, wobei durch den ersten Schneidblock ein erster Schnitt an einem ersten Körperteil (z.B. Tibia) durchgeführt werden soll und durch den zweiten Schneidblock ein Schnitt an einem zweiten Körperteil (z.B. Femur) durchgeführt werden soll. Das erste und das zweite Körperteil sind durch das Gelenk verbunden. Der Schnitt wird insbesondere durchgeführt, um ein Ende des ersten und zweiten Körperteils abzuschneiden, das dem Gelenk zugewandt ist, sich also insbesondere in der Nähe des Gelenks befindet. Die Schneidblöcke dienen der Führung eines Schneidmessers und dem Festlegen einer Ebene, in der der Schnitt mit dem Schneidmesser durchgeführt werden soll. Die Schneidblöcke umfassen insbesondere zwei Schneidführungsebenen, die bevorzugt parallel zueinander sind und zwischen denen die Klinge eines Schneidmessers geführt werden kann. Der Abstand der Schneidführungsebenen entspricht zumindest in etwa der Dicke der Klinge oder ist etwas größer. Die Schneidführungsebenen bestimmen somit die Lage der Schneidebene, in der der Schnitt mittels der Klinge erfolgt.

Die erfindungsgemäße chirurgische Verbindungsvorrichtung umfasst vorzugsweise einen ersten und zweiten Anbringabschnitt. Der erste Anbringabschnitt ist ausgebildet, um die chirurgische Verbindungsvorrichtung an dem ersten Schneidblock anbringen zu können. Der zweite Anbringabschnitt ist ausgebildet, um die chirurgische Verbindungsvorrichtung an dem zweiten Schneidblock anbringen zu können. Der erste und zweite Anbringabschnitt ist vorzugsweise zum Ausbilden einer mechanischen Verbindung zwischen der chirurgischen Verbindungsvorrichtung und dem jeweiligen Schneidblock ausgebildet. Die mechanische Verbindung ist vorzugsweise lösbar und sorgt vorzugsweise für eine ortsfeste Verbindung. Beispiele für eine mechanische Verbindung sind eine formschlüssige, kraftschlüssige oder stoffschlüssige Verbindung. Bevorzugt wird eine formschlüssige und/oder kraftschlüssige Verbindung mit Hilfe des Anbringabschnittes erzielt. Hierzu können an den Schneidblöcken auch besondere Kontaktabschnitte vorgesehen sein, mit denen die Anbringabschnitte eine lösbare mechanische Verbindung, insbesondere eine lösbare kraft- und/oder formschlüssige Verbindung eingehen, wenn sie die Kontaktelemente kontaktieren, um die chirurgische Verbindungsvorrichtung anzubringen. Die Verbindung kann beispielsweise passgenau sein und/oder eine rastende Verbindung sein. Abgesehen von gesonderten Kontaktabschnitten an den Schneidblöcken können die Anbringabschnitte auch so ausgebildet sein, dass sie in den Spalt zwischen den Schneidführungsebenen des Schneidblocks einbringbar sind, um mit den Schneidführungsebenen eine kraft- und/oder formschlüssige Verbindung einzugehen.

Weiter umfasst die chirurgische Verbindungsvorrichtung vorzugsweise einen Beabstandungsabschnitt, der den ersten Anbringabschnitt von dem zweiten Anbringabschnitt beabstandet. Auf diese Art und Weise ergibt sich eine definierte und feste Lagebeziehung zwischen dem ersten und zweiten Schneidblock, wenn beide Schneidblöcke mit der chirurgischen Verbindungsvorrichtung verbunden sind. Vorzugsweise ist der Abstand so gewählt, dass die chirurgische Verbindungsvorrichtung von einem Chirurgen, insbesondere auch mit Handschuhen greifbar ist. Insbesondere ist der Beabstandungsabschnitt derart ausgelegt, dass die Lagebeziehung (insbesondere der Abstand) zwischen dem ersten und zweiten Schneidblock (mechanisch) einstellbar ist, so dass die chirurgische Verbindungsvorrichtung vom Chirurgen an die jeweilige Körperstruktur angepasst werden kann. Auch kann hierzu eine Auswahl aus verschiedenen chirurgischen Verbindungsvorrichtungen mit verschiedenen fixen Abständen (z.B. 18 mm, 19 mm und 20 mm) zwischen dem ersten und zweiten Anbringabschnitt erfolgen. Insbesondere liegt die Untergrenze des Umfangs der Beabstandung bei 1 mm, vorzugsweise bei 1 cm. Vorzugsweise liegt die Obergrenze der Beabstandung bei 20 cm, mehr bevorzugt bei 10 cm, noch mehr bevorzugt bei 5 cm oder 3 cm. Ein typischer Abstand zwischen dem ersten und zweiten Anbringabschnitt liegt z.B. bei ca. 19 mm.
Ist der Beabstandungsabschnitt so gestaltet, dass mittels eines Verstellmechanismus der Abstand einstellbar ist. So umfasst der Beabstandungsabschnitt vorzugsweise zwei relativ zueinander bewegliche Teile, deren Relativlage durch ein Befestigungsmittel (z.B. Stellschraube, die durch Schlitze in den beweglichen Teilen hindurchgeht) oder eine Rastverbindung mechanisch feststellbar ist. Auf diese Art und Weise lässt sich dann der Abstand zwischen den Anbringabschnitten auf den gewünschten Abstand zumindest in einem vorbestimmten Umfang verändern. Der Rastmechanismus und/oder das oben genannte Befestigungsmittel zum Beispiel in Verbindung mit den Schlitzen bilden Beispiele für einen Verstellmechanismus, mit dem der Abstand zwischen dem ersten und zweiten Anbringabschnitt verstellbar ist.

Der Beabstandungsabschnitt ist insbesondere bevorzugt ausreichend groß ausgebildet, um einen Handgriff daran anbringen zu können.

Vorzugsweise ist zwischen dem ersten und zweiten Anbringabschnitt ein Zwischenraum vorgesehen, in dem Platz für die Schneidführungsebenen der Schneidblöcke und/oder für Kontaktabschnitte der Schneidblöcke vorhanden ist. Der Zwischenraum zwischen dem ersten und zweiten Anbringabschnitt ist deshalb vorzugsweise nicht vollständig ausgefüllt. Insbesondere ist vorzugsweise der erste Anbringabschnitt mit dem zweiten Anbringabschnitt nicht auf dem kürzesten Weg durch den Beabstandungsabschnitt verbunden. Vorzugsweise verläuft der Beabstandungsabschnitt zumindest teilweise und vorzugsweise zumindest überwiegend außerhalb des Zwischenraums zwischen dem ersten Anbringabschnitt und dem zweiten Anbringabschnitt. Der Zwischenraum zwischen dem ersten und zweiten Anbringabschnitt ist insbesondere derjenige Raum, der sich durch direkte und geradlinige Verbindung der Ränder des ersten Anbringabschnittes mit den Rändern des zweiten Anbringabschnittes ergibt.

Die Ränder der Anbringabschnitte umfassen Randabschnitte, wie ein Vorderende und ein Hinterende der Anbringabschnitte. Das Vorderende zeichnet sich dadurch aus, dass es bei dem Vorgang des Anbringen des Verbindungsabschnitts am Schneidblock vorangeht, also im Vergleich zu den übrigen Teilen der chirurgischen Verbindungsvorrichtung den kürzesten Abstand zum Schneidblock hat, wobei sich dieser Abstand immer weiter verringert, wenn die chirurgische Verbindungsvorrichtung dem Schneidblock immer näher gebracht wird, um die chirurgische Verbindungsvorrichtung mit dem Schneidblock zu verbinden. Gegenüberliegend des Vorderendes befindet sich das Hinterende. Das Hinterende zeichnet sich dadurch aus, dass es mit dem Beabstandungsabschnitt verbunden ist. Das Vorderende des Anbringabschnittes ist insbesondere als Kante der chirurgischen Verbindungsvorrichtung ausgebildet, die insbesondere ein Ende eines flächigen Abschnitts der Anbringvorrichtung bildet. Durch diese Anordnung des Beabstandungsabschnittes relativ zu den Anbringabschnitten ist eine definierte Beabstandung der Schneidblöcke ermöglicht. Außerdem wird verhindert, dass Teile des Schneidblockes ein Anbringen der Verbindungsvorrichtung an den Schneidblöcken behindern oder blockieren.

Gemäß einer weiteren Ausführungsform der vorliegenden Erfindung umfassen die Anbringabschnitte bewegliche Teile und Kontaktteile. Die Kontaktteile sind vorzugsweise ortsfest relativ zu dem Beabstandungsabschnitt. Die beweglichen Teile sind relativ zu den Kontaktteilen und insbesondere auch relativ zum Beabstandungsabschnitt vorzugsweise beweglich, insbesondere elastisch beweglich. Die beweglichen Teile können beispielsweise elastische Elemente wie Federn oder Gummi umfassen. Sie können aber auch elastisch mit einem Teil des Anbringabschnittes, insbesondere mit den Kontaktteilen verbunden sein. Beispielsweise kann es sich bei den beweglichen Teilen um (z.B. metallische oder Kunststoff-)Elemente handeln, die nur abschnittsweise, insbesondere nur an einem einzigen Abschnitt mit anderen Teilen des Anbringabschnittes insbesondere mit der den Kontaktteilen verbunden sind. Diese abschnittsweise Verbindung kann insbesondere elastisch ausgebildet sein. Insbesondere können die beweglichen Teile relativ zu den übrigen Teilen, insbesondere Kontaktteilen aus einer Ebene heraus gebogen sein. Wird auf die beweglichen Teile eine Kraft ausgeübt, um sie auf die Kontaktteile zu zu bewegen, so wirkt aufgrund der elastischen Ausbildung der beweglichen Teile eine elastische Kraft diesem entgegen. Die beweglichen Teile sind insbesondere elastisch relativ zu einer Ebene beweglich, die das Vorderende und Hinterende der Anbringabschnitte virtuell oder real verbindet und/oder in der die Kontaktteile liegen. Insbesondere erfolgt die Bewegung heraus aus der Ebene, also insbesondere quer zu der Ebene. Die Kontaktteile sind vorzugsweise flächig ausgebildet und liegen vorzugsweise in der vorgenannten Ebene oder die Oberflächen der Kontaktteile definieren die vorgenannte Ebene. Insbesondere weisen die Kontaktteile keine quer zur Ebene hervorragenden Fortsätze auf. Die flächige Ausbildung erlaubt den Kontaktteilen, in die Kontaktabschnitte der Schneidblöcke und insbesondere zwischen die Schneidführungsflächen eingeführt zu werden. Insbesondere können sie so ausgebildet sein, dass ein passgenaues Einführen ermöglicht wird.

Die beweglichen Teile sind vorzugsweise so ausgebildet, dass sich die aus der vorgenannten Ebene zwischen Vorderende und Hinterende herausragenden Kanten der beweglichen Teile in Richtung auf das Vorderende der Anbringabschnitte immer mehr der genannten Ebene annähern. Sie sind also vorzugsweise in Richtung auf das Vorderende abgeschrägt. Dies hat den Vorteil, dass bei Einbringen der Anbringabschnitte die beweglichen Teile stetig zunehmend in Richtung der Ebene gedrückt werden, falls der Anbringabschnitt in einen Spalt, z.B. Schneidführungsflächen oder in einen fugenförmigen Kontaktabschnitt eingeführt wird.

Vorzugsweise umfasst die chirurgische Verbindungsvorrichtung Anschlagabschnitte, die einer genauen Positionierung der chirurgischen Verbindungsvorrichtung relativ zu den Schneidblöcken dient. Die Anschlagabschnitte stehen hierzu vorzugsweise aus der vorgenannten Ebene zwischen Vorderende und Hinterende hervor, so dass sich bei Einführung des Anbringabschnittes in einen Spalt ein Anschlag der Spaltränder an den Anschlagabschnitten ergibt. Der Spalt wird beispielsweise zwischen den Schneidführungsebenen oder von dem Kontaktabschnitt des Schneidblocks gebildet. Vorzugsweise ist jeweils ein Anschlagabschnitt zwischen dem Beabstandungsabschnitt und einem der Verbindungsabschnitte vorgesehen.

Vorzugsweise umfasst die chirurgische Verbindungsvorrichtung eine Markervorrichtung. Mittels der Markervorrichtung kann die exakte Lage der chirurgischen Verbindungsvorrichtung detektiert werden. Da die relative Lage der chirurgischen Verbindungsvorrichtung relativ zu den Schneiddlöcken bekannt ist, insbesondere aufgrund des Anliegens der Schneidblöcke an den Anschlagabschnitten kann auch die Lage der Schneidblöcke durch die Detektion der Markervorrichtung, die an der chirurgischen Verbindungsvorrichtung angebracht ist, bestimmt werden.

Vorzugsweise können weitere chirurgische Instrumente oder chirurgische Vorrichtungen an der chirurgischen Verbindungsvorrichtung angebracht werden. Insbesondere kann ein Patella-Schneidapparat an der chirurgischen Verbindungsvorrichtung angebracht werden, um die Patella abschneiden zu können. Die Patella befindet sich zwischen Femur und Tibia. Da sich die chirurgische Verbindungsvorrichtung zwischen den beiden Schneidblöcken befindet, die einerseits dem Schneiden der Tibia und des Femurs dienen, befindet sie sich an einem geeigneten Platz, um die Patella zu schneiden. Umfasst die erfindungsgemäße chirurgische Verbindungsvorrichtung den Patella-Schneidapparat oder eine Anbringmöglichkeit für einen Patella-Schneidapparat, so ergibt sich automatisch eine geeignete Lage des Patella-Schneidapparats während der Operation.

Die Erfindung ist weiter auf ein chirurgisches System gerichtet, das sowohl die chirurgische Verbindungsvorrichtung als auch zwei Schneidblöcke umfasst, die ausgebildet sind, um mit der chirurgischen Verbindungsvorrichtung verbunden zu werden. Vorzugsweise umfasst das System auch einen Einstellmechanismus, der mit einem Schneidblock verbindbar ist oder verbunden ist. Der Einstellmechanismus wird vorzugsweise an einem Knochen, z.B. Tibia angebracht. An dem Einstellmechanismus sind vorzugsweise Verstellbedienteile angebracht, wie z.B. Räder, mit denen eine Lageänderung des mit dem Einstellmechanismus verbundenen Schneidblocks relativ zu den mit dem Knochen fest verbundenen Teil oder Teilen des Einstellmechanismus möglich ist. Welche Vorteile dies in Verbindung mit der erfindungsgemäßen chirurgischen Verbindungsvorrichtung hat, wird im Folgenden anhand eines erfindungsgemäßen Verfahrens beschrieben.

Wie gesagt, wird bevorzugt erst der Einstellmechanismus mit Befestigungsmitteln (z.B. Stiften oder Nägeln) an einer ersten Körperstruktur (z.B. Tibia) befestigt. Der Einstellmechanismus wird in einem nächsten Schritt mit dem ersten Schneidblock verbunden oder ist bereits mit dem ersten Schneidblock verbunden, wenn er an der ersten Körperstruktur befestigt wird. Eine Markervorrichtung ist vorzugsweise so an einem Bestandteil des Systems angebracht, dass durch den Einstellmechanismus bewirkte Lageänderungen durch Detektion der Markervorrichtung erfassbar sind. Die Markervorrichtung kann z.B. an dem Teil des Einstellmechanismus angebracht sein, der ortsfest mit der ersten Schneidvorrichtung verbunden ist oder verbindbar ist und der relativ zur ersten Körperstruktur durch Betätigung des Einstellmechanismus seine Lage ändert. Sie kann an dem ersten Schneidblock angebracht sein oder sie kann an der erfindungsgemäßen chirurgischen Verbindungsvorrichtung angebracht sein, wenn diese bereits mit dem ersten Schneidblock verbunden ist. Ist auch der zweite Schneidblock mit der chirurgischen Verbindungsvorrichtung bereits verbunden, so kann die Markervorrichtung auch am zweiten Schneidblock angebracht sein. Insbesondere ist in diesem Zustand mittels der Verbindungsvorrichtung eine ortsfeste Verbindung zwischen dem ersten und zweiten Schneidblock gegeben.

Durch Detektion der Markervorrichtung kann dann die Lage des zweiten Schneidblocks bestimmt werden. Durch Betätigen von Einstellmitteln am Einstellmechanismus kann diese Lage geändert werden. Die Markervorrichtung ist also relativ zu denjenigen Teilen des Einstellmechanismus ortsfest, für die eine Lageänderung relativ zum Knochen möglich ist. Sie ist insbesondere nicht ortsfest relativ zu denjenigen Teilen des Einstellmechanismus, die (ortsfest) relativ zur ersten Körperstruktur fixiert sind. Sie ist insbesondere ortsfest relativ zu dem Teil des Einstellmechanismus, der für eine ortsfeste Verbindung mit dem ersten Schneidblock ausgebildet ist und diese insbesondere eingegangen ist.

Ist die eben beschriebene Situation gegeben, so erfolgt vorzugsweise eine Einstellung der Lage des zweiten Schneidblocks relativ zu dem zweiten Körperteil (z.B. Femur), wobei die Einstellung mittels der Markervorrichtung und deren Detektion kontrolliert wird. Beispielsweise wird mittels eines Navigationssystems die Lage sowohl der ersten Körperstruktur als auch des Schneidblocks auf einem Monitor des Navigationssystems angezeigt. Das Navigationssystem umfasst insbesondere eine Datenverarbeitungsvorrichtung, die die Detektionssignale verarbeitet, die von einer Kamera in die Datenverarbeitungsvorrichtung eingegeben werden. Die Kamera detektiert die genannte Markervorrichtung.

Vorzugsweise wird von einem Zustand ausgegangen, bei dem die Verbindungsvorrichtung den ersten und zweiten Schneidblock verbindet. Außerdem ist vorzugsweise die Markervorrichtung ortsfest relativ zum ersten Schneidblock angebracht. Da eine Verbindung zwischen dem ersten und zweiten Schneidblock durch die chirurgische Verbindungsvorrichtung gegeben ist, ist in dem angegebenen Zustand auch eine ortsfeste Verbindung der Markervorrichtung relativ zum zweiten Schneidblock gegeben. Alternativ oder zusätzlich kann eine separate Markervorrichtung auch an dem zweiten Schneidblock angebracht sein.

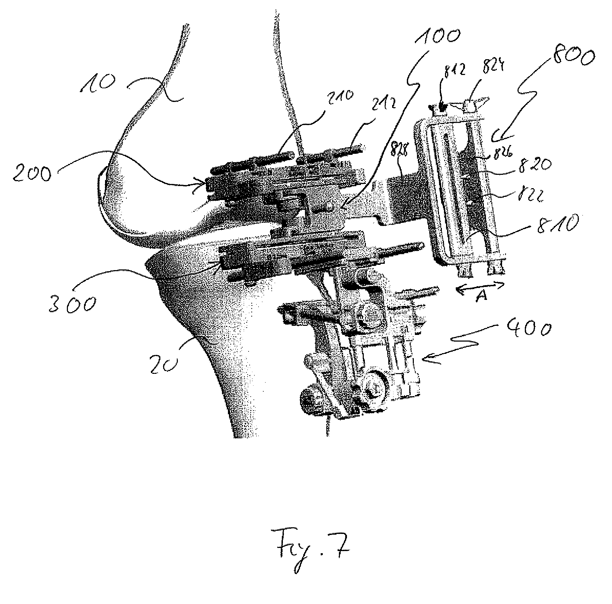

Im Folgenden wird eine erste Vorgehensweise zum Fixieren der beiden Schneidblöcke beschrieben. Eine zweite alternative Vorgehensweise wird in Verbindung mit der Figur 7 erläutert.

In einem ersten Schritt wird nun vorzugsweise mittels des Einstellmechanismus die Lage des zweiten Schneidblocks relativ zur zweiten Körperstruktur mittels einer mit dem zweiten Schneidblock ortsfest verbundenen Markervorrichtung, eingestellt. Ist die gewünschte Lage erreicht, so wird der zweite Schneidblock an der zweiten Körperstruktur fixiert. Nach der Fixierung wird die chirurgische Verbindungsvorrichtung entfernt, so dass der zweite Schneidblock relativ zum ersten Schneidblock beweglich ist. In einem zweiten Schritt wird dann mittels des Einstellmechanismus der erste Schneidblock relativ zur ersten Körperstruktur in die gewünschte Lage gebracht. Hierzu wird eine mit dem ersten Schneidblock ortsfest verbundene Markervorrichtung verwendet. Dann wird der erste Schneidblock an der ersten Körperstruktur fixiert. Somit sind sowohl der erste als auch der zweite Schneidblock jeweils an der ersten und zweiten Körperstruktur angebracht und genau platziert. In der nun gegebenen Lage kann der Schnitt an der jeweiligen Körperstruktur mithilfe der fixierten Schneidblöcke durchgeführt werden. Optional kann vor dem Schnitt der Einstellmechanismus von der ersten Körperstruktur entfernt werden. Vor Entfernung der chirurgischen Verbindungsvorrichtung kann auch eine Patella-Schneidvorrichtung daran angebracht werden. Alternativ kann die chirurgische Verbindungsvorrichtung bereits die Patella-Schneidvorrichtung umfassen. Das Patella-Schneiden wird dann vorzugsweise durchgeführt, bevor die chirurgische Verbindungsvorrichtung entfernt wird.

Die Erfindung bezieht sich weiter auf ein chirurgisches System mit der erfindungsgemäßen chirurgischen Verbindungsvorrichtung, auf ein Navigationssystem mit dem chirurgischen System und auf die Verwendung der chirurgischen Verbindungsvorrichtung, des chirurgischen Systems und/oder des Navigationssystems.

Bei der folgenden detaillierten Beschreibung werden weitere Vorteile und Merkmale der Erfindung offenbart. Verschiedene Merkmale und unterschiedliche Ausführungsformen können miteinander kombiniert werden.
Figur 1 zeigt ein Navigationssystem der vorliegenden Erfindung mit Navigationsvorrichtung und einem chirurgischen System gemäß der vorliegenden Erfindung;
Figur 2 zeigt ein chirurgisches System gemäß der vorliegenden Erfindung, wobei die erfindungsgemäße Verbindungsvorrichtung gerade entfernt wird;
Figur 3 zeigt zwei nicht verbundene am Knochen fixierte Schneidblöcke;
Figur 4 zeigt eine Ausführungsform der erfindungsgemäßen chirurgischen Verbindungsvorrichtung;
Figur 5 zeigt eine weitere Ausführungsform der erfindungsgemäßen chirurgischen Verbindungsvorrichtung;
Figur 6 zeigt eine erfindungsgemäße chirurgische Verbindungsvorrichtung mit Markervorrichtung;
Figur 7 zeigt ein erfindungsgemäßes chirurgisches System mit Patella-Schneidvorrichtung gemäß einer ersten Ausführungsform;
Figuren 8 bis 12 zeigen eine Patella-Schneidvorrichtung gemäß einer zweiten Ausführungsform, die mit der chirurgischen Verbindungsvorrichtung beispielhaft verbunden ist.

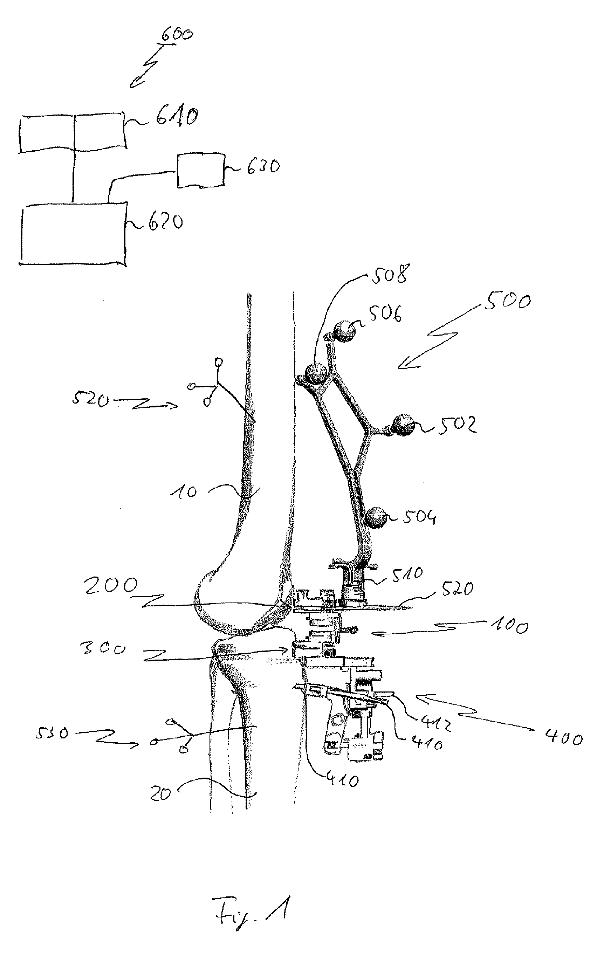

Figur 1 zeigt ein chirurgisches System gemäß der vorliegenden Erfindung mit den Bestandteilen 100, 200, 300, 400 und 500, die im Folgenden näher erläutert werden. Das System umfasst die erfindungsgemäße chirurgische Verbindungsvorrichtung 100. Figur 1 zeigt weiter einen Femur 10 und eine Tibia 20 und eine Navigationsvorrichtung 600. Erfindungsgemäß ist vorzugsweise nur ein einziger Einstellmechanismus 400 vorgesehen und über Stifte 410 und 412 an der Tibia 20 angebracht. Dieser einzige Einstellmechanismus genügt aufgrund der Verwendung der chirurgischen Verbindungsvorrichtung 100, die einen Tibia-Schneidblock 300 (erster Schneidblock) mit einem Femur-Schneidblock 200 (zweiter Schneidblock) verbindet, zum Einstellen der fixierten Lage beider Schneidblöcke. Mit dem Femur-Schneidblock 200 ist ein Referenzstern 500 ortsfest verbunden. Bei der gezeigten Ausführungsform ist der Referenzstern 500 lösbar mit einer Schnappverbindung 510 mit einer Plattform 520 verbunden, die wiederum mit dem zweiten Schneidblock 200 verbunden ist. Beispielsweise kann die Plattform 520 zwischen die Schneidführungsflächen des Schneidblockes eingeführt sein. Insbesondere umfasst sie Anschlagpunkte, um eine definierte relative Lage des Referenzsterns 500 zum Schneidblock 200, insbesondere den Schneidführungsebenen und/oder eine definierte relative Lage der Schneidebene relativ zum Referenzstern zu haben. Um die relative Lage möglichst genau zu bestimmen sind vorzugsweise Anschlagpunkte oder Einrastelemente an der Plattform 520 vorgesehen, die zur Anlage an oder zum Eingriff mit dem Schneidblock gebracht werden können. Auch kann die Plattform 520 so ausgebildet sein, dass sie möglichst passgenau zwischen die Schneidführungsebenen in den Schneidblock (200 oder 300) einführbar ist. Somit ist eine definierte relative Lage zwischen den Schneidführungsebenen und dem Referenzstern gegeben. Die relative Lage ist der Navigationsvorrichtung 600 bekannt und dort abgespeichert.

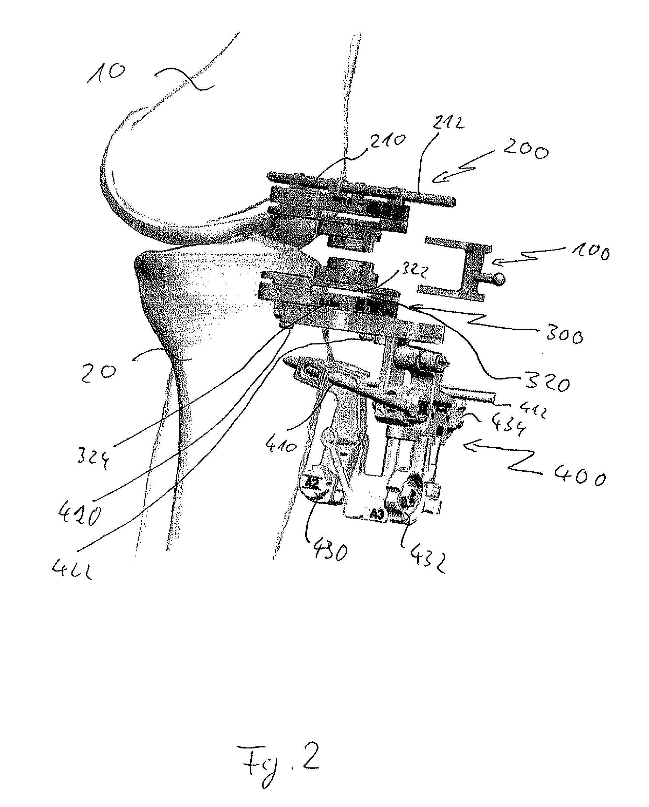

Der Referenzstern 500 umfasst z.B. Markerelemente 502, 504, 506 und 508, die insbesondere kugelförmig ausgebildet sein können. Die Markerelemente können aktiv oder passiv ausgebildet sein. Aktive Markerelemente senden aktiv Strahlung oder Wellen aus. Passive Markerelemente reflektieren Strahlung oder Wellen. Bei der Strahlung kann es sich insbesondere um elektromagnetische Strahlung, wie UV-Licht, sichtbares Licht oder Infrarotlicht handeln. Bei den Wellen kann es sich insbesondere um Ultraschallwellen handeln. Die Markerelemente haben vorzugsweise eine vordefinierte Lage relativ zueinander, die typisch für den Referenzstern 500 ist, so dass der Referenzstern 500 durch eine Navigationsvorrichtung 600 erkannt werden kann. Die Navigationsvorrichtung 600 umfasst insbesondere eine Detektionseinrichtung 610 bestehend beispielsweise aus zwei Kameraelementen. Weiter umfasst sie vorzugsweise eine Datenverarbeitungseinrichtung 620, die die Detektionssignale der Detektionseinrichtung 610 verarbeitet, um daraus insbesondere Lagedaten des Referenzsterns 500 zu ermitteln. Dies erlaubt aufgrund der bekannten relativen Lage zwischen Referenzstern 500 und der Schneidebene, die Lage der Schneidebene in einem von der Navigationsvorrichtung genutzten Bezugssystem zu berechnen. Vorzugsweise ist in den betreffenden Körperteilen, also beispielsweise den Femur 10 ebenfalls ein Referenzstern 520 und in der Tibia ein Referenzstern 530 vorgesehen. Vorzugsweise ist die relative Lage eines dreidimensionalen Modells des Körperteils (Femur oder Tibia) relativ zu dem am Körperteil fixierten Referenzstern (520 oder 530) der Navigationsvorrichtung bekannt und insbesondere dort abgespeichert. Deshalb ist die Bestimmung der Lage der Schneidebene relativ zu dem jeweiligen Körperteil, also beispielsweise relativ zu dem Femur 10 oder der Tibia 20 mittels der Datenverarbeitungsvorrichtung 620 möglich. Dann kanndie Datenverarbeitungsvorrichtung 620 das berechnete Ergebnis, insbesondere die relative Lagebeziehung zwischen einem Modell des Körperteils (Femur und/oder Tibia) relativ zu der Schneidebene auf einem Monitor 630 anzeigen bzw. ein Signal an dem Monitor 630 senden, woraufhin die genannte relative Lagebeziehung auf dem Monitor 630 angezeigt wird. Auf diese Art und Weise kann ein Chirurg durch Verstellung des Einstellmechanismus 400 die Lage der Schneidebene ändern und die Lage der Schneidebene an dem Monitor 620 überprüfen. Falls der Chirurg die Lage der Schneidebene relativ zu dem Femur 10 insbesondere aufgrund der Anzeige auf dem Monitor 630 als passend oder der Planung entsprechend ansieht, so kann dann der Schneidblock 200 am Femur 10 mittels in der Figur 1 nicht zu sehender Stifte befestigt werden. Wie dies dann nach Entfernung des Referenzsterns 500 und der chirurgischen Verbindungsvorrichtung 100 aussieht, ist der Figur 2 zu entnehmen. Dort sind Stifte 210 und 212 gezeigt, die in den Femur 10 eingeführt sind, um den Schneidblock 200 am Femur in der gewünschten Lage zu befestigen. Die Verbindungsvorrichtung 100 wird gerade entfernt.

Nach Entfernung der Verbindungsvorrichtung 100 wird dann vorzugsweise die Lage des ersten Schneidblocks 300 eingestellt. Hierzu wird vorzugsweise wiederum ein Referenzstern genutzt, beispielsweise der Referenzstern 500, der in Figur 1 gezeigt ist. Der Referenzstern, der vorzugsweise an einer Plattform befestigt ist (z.B. der Plattform 520) wird mit der Schneidvorrichtung 300 so verbunden, dass sich vorzugsweise eine definierte (und der Navigationsvorrichtung 600 bekannte) Lage der Schneidebene des Schneidblocks 300 relativ zum Referenzstern 500 ergibt. Hierzu kann beispielsweise die genannte Plattform 520 in den Spalt 320 zwischen den Schneidführungsebenen 322 und 324 eingeführt werden. Die Plattform ist vorzugsweise passgenau für den Spalt 320 ausgebildet, so dass sich kein Verkippen der Plattform relativ zur Schneidebene ergeben kann. Alternativ können natürlich andere Verbindungsmöglichkeiten zur Verbindung des Referenzsterns mit einem der Schneidblöcke 200, 300 verwendet werden, wie dies auch oben ausgeführt wurde. Insbesondere können am Schneidblock 200, 300 Rastverbindungen vorgesehen sein, so dass insbesondere ein Einführen der Plattform 520 in den Spalt 320 nicht erforderlich ist sondere eine Verbindung zwischen Referenzstern 500 und Schneidblock 200, 300 außerhalb des Spaltes 320 erfolgt.

Der Schneidblock 300 ist mit dem Einstellmechanismus 400 vorzugsweise mechanisch und vorzugsweise lösbar verbunden. Insbesondere sind vorzugsweise an dem Einstellmechanismus 400 Befestigungsmittel 420 und 422 vorgesehen. Durch Lösen dieser Befestigungsmittel 420, 422 (z.B. Schrauben) lässt sich dann der Einstellmechanismus 400 von dem Schneidblock 300 entfernen, falls dies erwünscht ist. Mehrere Einstellschrauben 430, 432, 434 etc. sind am Einstellmechanismus 400 vorgesehen, um die Lage des Schneidblocks 300 relativ zu den Stiften 412 und 410, mit der der Einstellmechanismus 400 an der Tibia 20 befestigt ist, ändern zu können. Die Lageänderung erfolgt auch in diesem Fall solange, bis die gewünschte Lage des Schneidblocks 300, also die gewünschte Lage der Schneidebene des Schneidblocks 300 relativ zur Tibia 20 erzielt worden ist.

Durch die chirurgische Verbindungsvorrichtung 100 kann demnach der Einstellmechanismus 400 doppelt genutzt werden. Zum einen kann er zum Einstellen der Lage des Schneidblocks 200 verwendet werden und danach auch noch zur Einstellung der Lage des Schneidblocks 300. Dadurch muss nur ein Einstellmechanismus 400 am Knochen befestigt werden und die Schäden am Knochen werden somit verringert.

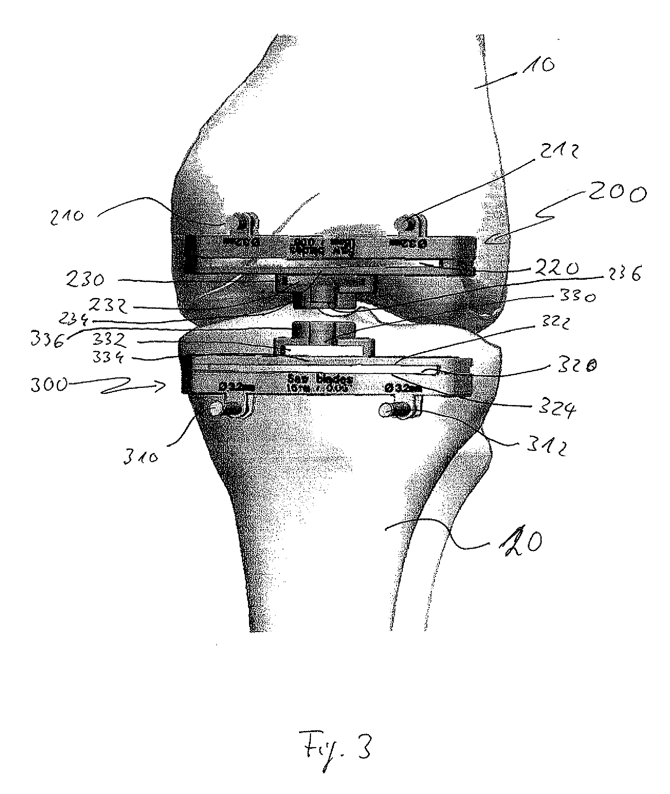

Die Situation nach Entfernung der chirurgischen Verbindungseinrichtung 100 und auch des Einstellmechanismus 400 sowie des in Figur 2 nicht gezeigten Referenzsterns 500 ist in Figur 3 gezeigt. Gleiche Bezugszeichen bezeichnen gleiche Teile. Dies gilt auch für die übrigen Abbildungen.

In dem in Figur 3 gezeigten Zustand ist der Tibia-Schneidblock 300 an der gewünschten Position durch Stifte 310, 312 an der Tibia 20 befestigt.

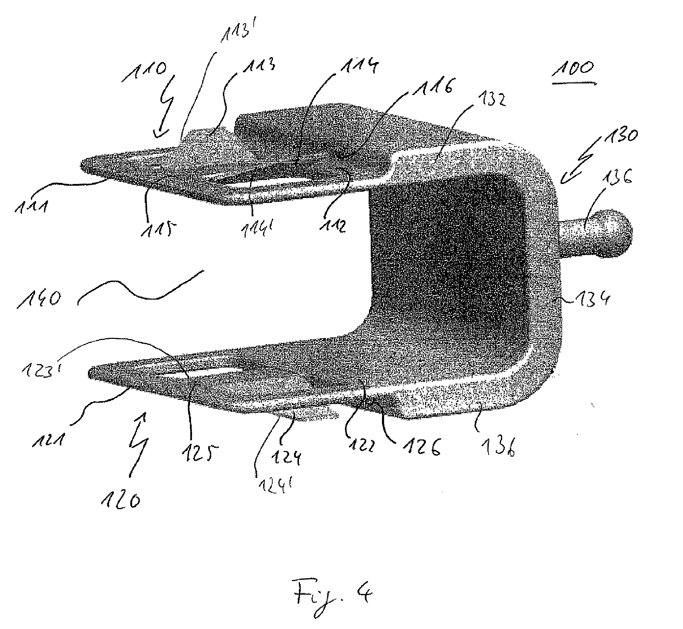

Den Aufbau einer erfindungsgemäßen chirurgischen Verbindungsvorrichtung 100 zeigt die Figur 4. Die chirurgische Verbindungsvorrichtung 100 umfasst Vorderenden 111 und 121, die jeweils in Kontaktspalte 232 und 332 von Kontaktabschnitten 230 und 330 einführbar sind.

Die Dicke der Vorderenden und insbesondere der Kontaktteile 115 und 125 ist vorzugsweise kleiner oder gleich der Spaltdicke der Kontaktspalte 230 und/oder 330. Vorzugsweise sind die Kontaktteile 115 und 125 passgenau zu den Kontaktspalten 230 und 330 ausgebildet. Die Kontaktteile sind vorzugsweise flächig ausgebildet. In dem dem Vorderende 111 abgewandten Abschnitt 112 des Kontaktteils 115 ist der Kontaktteil vorzugsweise mit dem Beabstandungsabschnitt 130 verbunden, vorzugsweise einstückig verbunden. Das Kontaktteil 115 kann in dem Bereich 112 aber auch mit Befestigungsmittel an dem Beabstandungsabschnitt 130 angebracht sein. In der gezeigten Ausführungsform ist das Kontaktteil 115 flächig ausgebildet. Relativ zu dem Kontaktteil 115 sind bewegliche Teile 113 und 140 beweglich. Diese beweglichen Teile sind in der gezeigten Ausführungsform an dem Kontaktteil 115 angeformt, insbesondere einstückig dazu ausgebildet. Aufgrund der Materialausbildung (z.B. Metall oder Kunststoff) sind die beweglichen Teile durch Einwirkung einer Kraft relativ zum Kontaktteil beweglich, insbesondere elastisch beweglich. Das bedeutet, dass sie nach Ende der Krafteinwirkung wieder von selbst in eine Ausgangsposition zurückkehren. Dies wird insbesondere zu einer kraftschlüssigen Verbindung der chirurgischen Verbindungsvorrichtung 100 mit dem Kontaktabschnitt 230 und/oder 330 genutzt. Die beweglichen Teile 113 und 114 drücken somit im eingeführten Zustand der chirurgischen Verbindungsvorrichtung gegen die Begrenzung des Kontaktspaltes 232. Analog drücken bewegliche Teile 124 und 123 (nicht gezeigt) gegen eine Begrenzungswand des Kontaktspalts 332. Das Kontaktteil 125 wird dabei in den Kontaktspalt 332 eingeführt. Am Hinterende 112 und 122 der Kontaktteile 115 und 125 befindet sich vorzugsweise ein Anschlag 116 und 126, der eine Kante des Beabstandungsabschnittes bildet und somit zwischen Anbringabschnitt und Beabstandungsabschnitt angeordnet ist. An dem Anschlag 116 bzw. 126 liegt nach vollendetem Einführen der chirurgischen Verbindungsvorrichtung in die Kontaktabschnitte 230 und 330 eine Vorderkante 234 bzw. 334 des Kontaktabschnitts 230 bzw. 330 an. Diese Vorderkante ist jeweils insbesondere eine Vorderkante des Schneidebenenspalts 220 und 320 bei der in Figur 3 gezeigten Ausführungsform.

Die chirurgische Verbindungsvorrichtung 100 hat vorzugsweise einen U-förmigen Querschnitt, wie dies aus Figur 4 erkennbar ist. Die chirurgische Verbindungsvorrichtung hat in der gezeigten Ausführungsform die Form eines dreidimensional geformten Buchstaben U. Die freien Enden des 3D-Buchstabens U werden in die Kontaktspalte 230 und 330 eingeführt. Zwischen dem Kontaktteil 115 und 125 befindet sich ein Zwischenraum 140. Dieser Zwischenraum bietet Platz für Teile 236 und 336 des Kontaktabschnittes 230 und 330, wobei die Teile 236 und 336 sich gegenüberliegen, wenn die Schneidblöcke 200 und 300 durch die chirurgische Verbindungsvorrichtung verbunden sind.

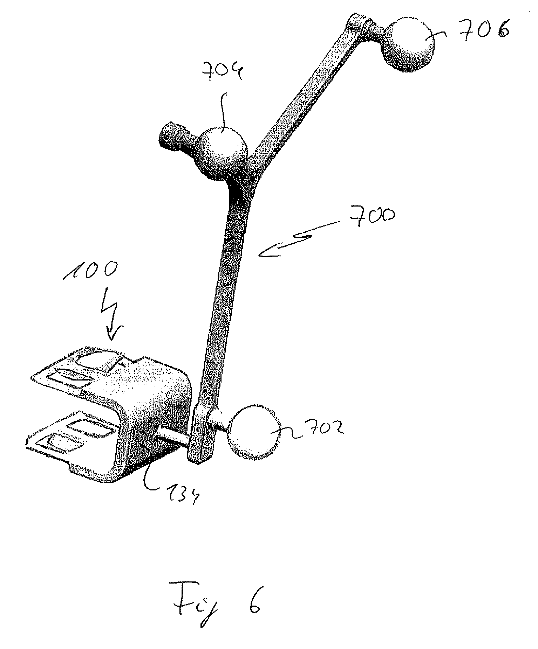

Der Beabstandungsabschnitt 130 kann in drei Abschnitte 132, 134 und 136 unterteilt werden, die vorzugsweise einstückig miteinander verbunden sind. Die Abschnitte 132 und 136 sind jeweils Bestandteile der Arme des 3D-Buchstabens U. Der Abschnitt 134 bildet den Boden des 3D-Buchstabens U. An dem Bodenabschnitt 134 kann beispielsweise ein Handgriff 136 angebracht sein. Dieser Handgriff kann natürlich auch an den Abschnitten 132 und 136 angebracht sein. Bevorzugt wird jedoch aufgrund der besseren Erreichbarkeit der Abschnitt 134. An dem Beabstandungsabschnitt 130 und insbesondere an dessen Bodenteil 134, der die Arme des 3D-Buchstabens U verbindet, können auch andere Vorrichtungen, insbesondere ein Referenzstern (siehe Figur 6) oder eine Patella-Schneidvorrichtung (siehe Figur 7) angebracht sein, wie dies später noch erläutert wird.

Die beweglichen Teile 113, 114, 123 (nicht gezeigt) und 124 weisen jeweils eine sich in Richtung auf das Vorderende verjüngende Kante 113', 114', 123' (nicht gezeigt) und 124' auf. Die Kante verjüngt sich in Richtung auf das Vorderende und nähert sich dabei der außen liegenden Oberfläche der Kontaktteile 115 und 125 an. Durch diese Verjüngung wird beim Einführen der chirurgischen Verbindungsvorrichtung 100 in die Kontaktabschnitte 230 und 330 der Schneidblöcke 200 und 300 die beweglichen Teile 113, 114, 123 (nicht gezeigt) und 124 jeweils in Richtung auf die Fläche der Kontaktteile 115 und 125 mehr und mehr gedrückt, bis die beweglichen Teile 113, 114, 123 (nicht gezeigt) und 124 mit der Fläche der Kontaktteile 115 und 125 ausgerichtet sind. Aufgrund ihrer elastischen Bewegbarkeit drücken die beweglichen Teile im Zustand der Verbindung mit den Schneidblöcken 200, 300 gegen eine Wand des Schneidspaltes und sorgt somit für eine kraftschlüssige Verbindung. Die beweglichen Teile 113, 114, 123 (nicht gezeigt) und 124 können insbesondere durch einen Stanzvorgang aus einem gemeinsamen flächigen Abschnitt herausgestanzt werden, der jeweils ein Kontaktteil und die zugeordneten beweglichen Teile umfasst. Die beweglichen Teile werden dann aus der Ebene, die die Kontaktteile bilden, heraus gebogen, um so später für eine kraftschlüssige Verbindung sorgen zu können.

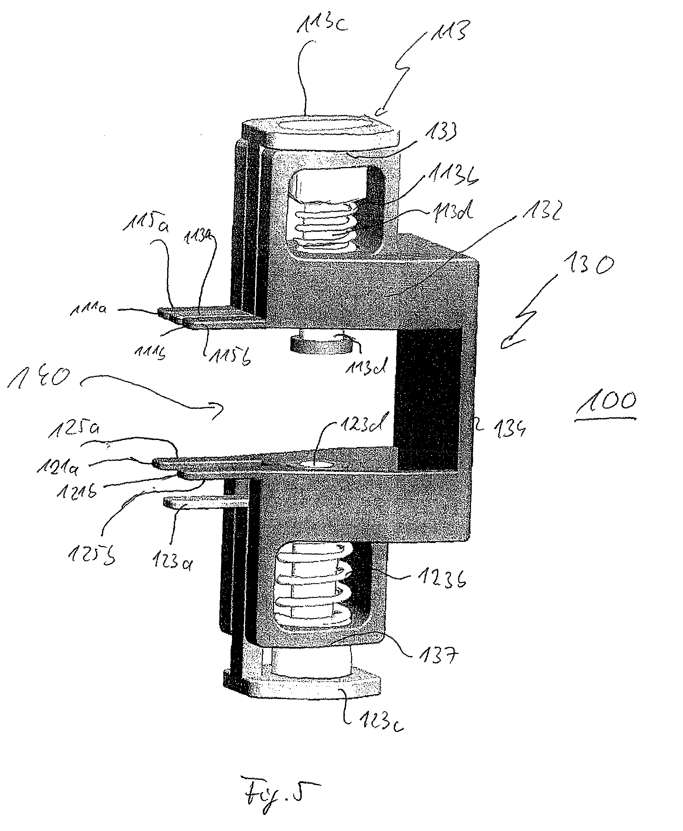

Figur 5 zeigt eine weitere Ausführungsform einer chirurgischen Verbindungsvorrichtung 100. Teile mit gleicher oder ähnlicher Funktion sind mit denselben Bezugszeichen bezeichnet. Ein Beabstandungsabschnitt 130 hat wiederum eine U-förmige Gestalt. Kontaktteile 115a und 115b an einem Arm und 125a und 125b an einem anderen Arm der U-förmigen Verbindungsvorrichtung sind wiederum ortsfest relativ zu dem Beabstandungsabschnitt 130. Vorderenden 111a, 111b und 121a und 121b der Kontaktteile sind ausgebildet, um in die Kontaktspalte eingeführt zu werden. Ein bewegliches Teil 113 umfasst ein Einführteil 113a, das in eine Ebene bewegt werden kann, in der die Kontaktteile 115a und 115b liegen. In der in Figur 5 gezeigten Situation liegt das Einführteil 113a in derselben Ebene wie die Kontaktteile 115a und 115b. Das bewegliche Teile 113 umfasst weiter eine Feder 113b, eine Betätigungstaste 113c mit einem Zylinder 113d. Die Feder ist gegenüber dem Beabstandungsabschnitt 130, genauer gegenüber dem Teil 132 des Beabstandungsabschnittes 130 abgestützt. Bewegt man die Taste 113c nach unten, also in Richtung auf den Beabstandungsabschnitt 130, so muss man gegen die Kraft der Feder 113b arbeiten, die am Teil 132 abgestützt ist. Dabei wird der Zylinder 113d nach unten gedrückt, bis er die in Figur 5 sichtbare Lage einnimmt. Wenn der Druckknopf 113c gegen einen Anschlag 133 des Beabstandungsabschnitts trifft, dann befindet sich auch der Einführabschnitt 113a in derselben Ebene wie die Kontaktteile 115a und 115b.

Der untere Teil der Figur 5 zeigt den nicht gedrückten Zustand eines Betätigungsknopfes 123c. Deshalb ist der Einführabschnitt 123a auch nicht in derselben Ebene, wie die Kontaktteile 125a und 125b. Die Feder 123b ist im entspannten Zustand. Deshalb ist der Zylinder 123d im Gegensatz zum Zylinder 113d nicht vorstehend in den Zwischenraum, der von dem U-förmigen Beabstandungsabschnitt 130 umgeben wird. Ein Drücken der Taste 123c, bis die Fläche der Taste 123c gegen einen Anschlag 137 des Beabstandungsabschnittes 130 stößt, bewirkt, dass der Einführabschnitt 123a in derselben Ebene liegt wie die Kontaktteile 125a und 125b.

Sind die Tasten 113c und 123c also bis zum Anschlag gedrückt, so kann die chirurgische Verbindungsvorrichtung in die Kontaktspalte zweier Schneidblöcke eingeführt werden. Werden die Tasten 113c und 123c nach dem Einführen losgelassen, so ergibt sich eine kraftschlüssige Verbindung. Die beiden Schneidblöcken 200, 300 befinden sich also im verbundenen und somit relativ zueinander im ortsfesten Zustand. Letzteres ist Voraussetzung, um mit einem einzigen Einstellmechanismus 400 die gewünschte Position zweier Schneidblöcke 200, 300 einstellen zu können.

Figur 6 zeigt eine chirurgische Verbindungsvorrichtung 100 mit einem daran angebrachten Referenzstern 700 mit Markerelementen 702, 704 und 706. Der Referenzstern 700 ist am Bodenteil 134 der U-förmigen chirurgischen Verbindungsvorrichtung 100 angebracht. Mithilfe des Referenzsterns 700 kann man es auch bei schwierigen Sichtverhältnissen dem Operateur ermöglichen, die chirurgische Verbindungsvorrichtung 100 mit den Kontaktabschnitten 230, 330 der Schneidblöcke 200, 300 zu verbinden. Außerdem kann der Referenzstern 700 zusätzlich zu diesem Zweck (oder auch ausschließlich) als Navigationshilfe zum Platzieren des zweiten Schneidblocks 200 verwendet werden, da eine ortsfeste Verbindung zwischen dem Schneidblock (insbesondere zweiten Schneidblock 200) und dem Referenzstern 700 gegeben ist.

Figur 7 zeigt die Situation bei fixiertem Femur-Schneidblock 200. Der Schneidblock 300 ist ebenfalls durch Stifte fixiert. Die Figur 7 betrifft eine alternative zweite Vorgehensweise, zu der bereits oben beschriebenen Vorgehensweise. Demnach wird mithilfe des Einstellmechanismus 400 zuerst die Lage des Tibia-Schneidblocks 300 eingestellt. Dann wird der Tibia-Schneidblock 300 fixiert. Anschließend wird die Lage des Femur-Schneidblocks 200 relativ zum Femur durch Verlagerung der Tibia und des Femurs eingestellt und der Femur-Schneidblock 200 wird dann fixiert. Während der Fixierung des Femur-Schneidblocks ist dieser insbesondere über die Verbindungsvorrichtung 100 mit dem Tibia-Schneidblock 300 ortsfest verbunden. Bei der alternativen Vorgehensweise gemäß Figur 7 wird demnach der Einstellmechanismus 400 nur zur Einstellung der Lage des Tibia-Schneidblocks 300 genutzt. Der Abstand zwischen dem Tibia-Schneidblock 300 und dem Femur-Schneidblock 200 wird durch Auswahl einer geeigneten Verbindungsvorrichtung oder durch Einstellung des Abstandes zwischen den Schneidblöcken mittels des oben beschriebenen Verstellmechanismus bestimmt. Die Lage des Femur-Schneidblocks 200 relativ zu dem Femur kann dann durch Verlagerung des Femurs und/oder der Tibia erfolgen. Die Einstellung der Lage des Femur-Schneidblocks 200 relativ zum Femur durch Bewegung der Tibia 20 und des Femurs 10 kann auch bei der oben bereits beschriebenen ersten Vorgehensweise genutzt werden, um insbesondere eine grobe Einstellung vorzunehmen. Eine Feineinstellung kann bei der ersten Vorgehensweise mittels des Einstellmechanismus erfolgen.

Auch der Einstellmechanismus 400 ist an der Tibia 20 befestigt. Mit der Verbindungsvorrichtung 100 verbunden ist eine Patella-Schneidvorrichtung 800. In dem gezeigten Fall ist die Patella-Schneidvorrichtung 800 einstückig mit der chirurgischen Verbindungseinrichtung 100 verbunden. Die Patella-Schneidvorrichtung 800 umfasst einen verstellbaren Schneidschlitzteil 810 und zwei Dornfortsätze 820, 822 zum Fixieren der Patella. Der Schneidschlitz 810 ist durch eine Schraube 812 in die mit A bezeichneten Richtungen verstellbar. Ebenfalls ist mit einer Schraube 824 der Abstand zu einer hinteren Anlagefläche 826 relativ zu der vorderen Anlagefläche 828 verstellbar. Die vordere Anlagefläche 828 ist ortsfest mit der Verbindungsvorrichtung 100 verbunden. Durch Verstellen der hinteren Anlagefläche 826 kann die Patella zwischen der vorderen Anlagefläche 828 und der hinteren Anlagefläche 826 eingeklemmt werden.

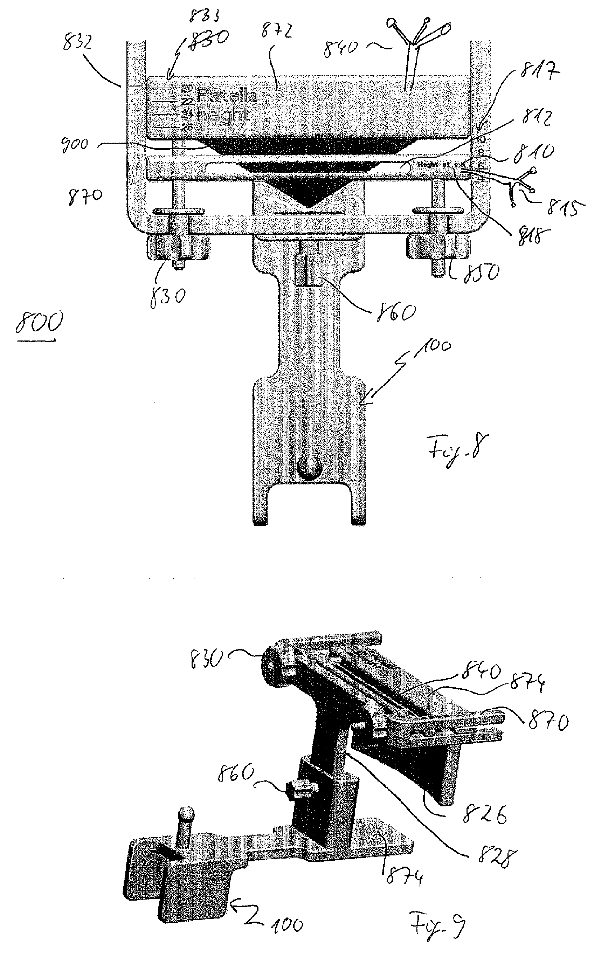

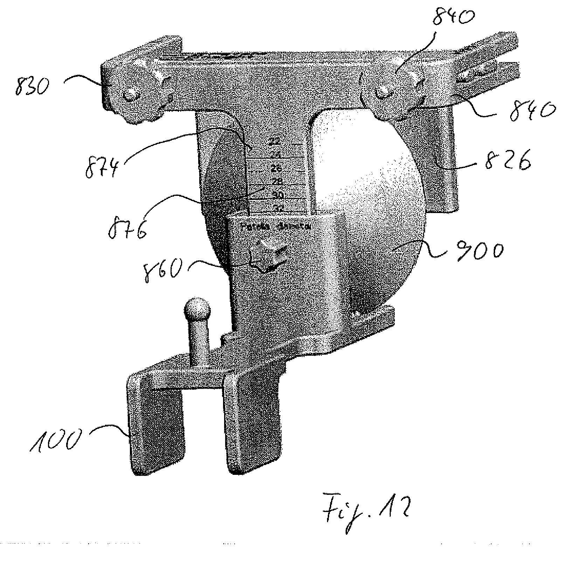

Figur 8 zeigt eine alternative Ausführungsform der Patella-Schneidvorrichtung 800. Der Abstand zwischen einer hinteren Anlagefläche 826 und einer vorderen Anlagefläche 828 (siehe Figur 9) lässt sich mittels einer Schraube 830 einstellen. Ist die Patella zwischen der vorderen und hinteren Anlagefläche eingeklemmt, so lässt sich mittels einer Skala 830 und einem Skalenstrich 832 die Patellahöhe messen. Die hintere Anlagefläche 826 ist beweglich relativ zur vorderen Anlagefläche und relativ zu einem Rahmen 870. Gemäß einer Ausführungsform ist an der hinteren Anlagefläche 826 oder an einem damit ortsverbundenen Teil, beispielsweiseder Skala 833, ein Referenzstern 840 angebracht. Durch Ablesen der Lage des Referenzsterns 840 lässt sich somit von einem Navigationssystem (das beispielsweise die Navigationsvorrichtung 600) automatisch die Patellahöhe bestimmen, ohne dass eine Ablesen der Skala 833 mit dem Skalenstrich 832 notwendig wäre. Vorzugsweise aber nicht obligatorisch umfasst die Patella-Schneidvorrichtung die erfindungsgemäße Verbindungsvorrichtung 100. Die Patella-Schneidvorrichtung 800 kann also auch ohne der erfindungsgemäßen Verbindungsvorrichtung 100 eingesetzt werden. Insbesondere betrifft die Patella-Schneidvorrichtung 800 ohne Verbindungsvorrichtung 100 eine weitere Erfindung. Diese Patella-Schneidvorrichtung umfasst also ein Einstellmittel 830 (beispielsweise Drehknopf 830) um den Abstand zwischen der vorderen und hinteren Anlagefläche einzustellen. Sie umfasst weiter vorzugsweise ein Einstellmittel (beispielsweise Einstellrad 850) um die Schneidhöhe einzustellen. Das Einstellrad 850 ist mechanisch mit einem Schneidschnitzteil 810 verbunden, das einen Schneidschlitz 812 zum Einführen einer Klinge zum Schneiden der Patella umfasst. Ebenfalls kann an dem Schneidschnitzteil 810 ortsfest ein Referenzstern 815 angebracht sein. Durch Detektion des Referenzsterns 815 mittels der Navigationsvorrichtung 600 kann die eingestellte Schneidhöhe bestimmt werden. Alternativ kann durch Ablesen der Skala 817 und des Skalenstriches 818 die Schneidhöhe abgelesen werden (siehe Figur 12).

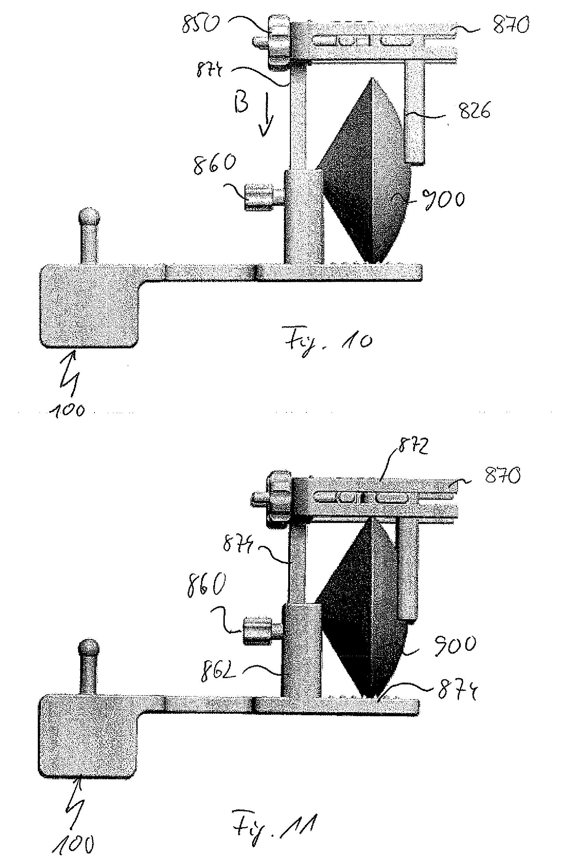

Ein Verstellteil 860 erlaubt die Anpassung von Patellaanlageflächen an den Durchmesser der Patella, wie dies in Figuren 10 und 11 gezeigt ist. Ein oberer Rahmen 870 lässt sich in die mit B bezeichnete Richtung absenken. Die abgesenkte Situation ist in Figur 11 gezeigt. In dieser Situation kann dann mit dem Drehknopf 860 der Rahmen 870 fixiert werden. Figur 12 zeigt eine weitere Ansicht der Patella-Schneidvorrichtung 800. Der Rahmen 870 befindet sich in seiner abgesenkten Stellung. Dies bedeutet, dass der volle Durchmesser der Patella 900 zwischen einem Teil 872 des Rahmens 870 und einer vorzugsweise mit Noppen versehenen Auflagefläche 874 liegt. Die Auflagefläche 874 ist vorzugsweise ortsfest mit einem Standfuß 862 verbunden. Der Steg 874 des Rahmens 870 ist insbesondere in die Richtung B (siehe Figur 10) beweglich. Der Steg 874 wird relativ zum Standfuß 862 durch das Einstellrad 860 fixiert. Der Standfuß 862 ist in der Figur 11 gezeigten Ausführungsform ortsfest zu der Verbindungsvorrichtung 100. Wie in Figur 12 gezeigt ist, lässt sich durch eine Skala 876 der Patelladurchmesser ablesen, wenn das Teil 872 des Rahmens 870 und die Auflagefläche 874 jeweils mit dem Rand der Patella 900, insbesondere an gegenüberliegenden Stellen in Kontakt sind. Der Rand der Patella 900 ist typischerweise oval oder kreisförmig und verläuft entlang der Außenoberfläche der Patella 900, dort wo diese den größten Umfang hat.

## Patentansprüche

1. Chirurgische Verbindungsvorrichtung (100) zum Verbinden eines ersten (300) und zweiten (200) Schneidblocks, die jeweils der Durchführung eines Schnitts an einer ersten (20) und zweiten (10) anatomischen Struktur dienen,
mit einem ersten Anbringabschnitt (120) zum Anbringen der chirurgischen Verbindungsvorrichtung (100) an dem ersten Schneidblock (300) durch mechanische Verbindung und
mit einem zweiten Anbringabschnitt (110) zum Anbringen an dem zweiten Schneidblock (200) durch eine lösbare mechanische Verbindung,
mit einem Beabstandungsabschnitt (130), der den ersten Anbringabschnitt (120) von dem zweiten Anbringabschnitt (110) beabstandet.

2. Chirurgische Verbindungsvorrichtung (100) nach Anspruch 1,
bei welcher der Beabstandungsabschnitt (130), um die Beabstandung zu erzielen, den ersten Anbringabschnitt (120) mit dem zweiten Anbringabschnitt (110) außerhalb eines Zwischenraums zwischen dem ersten und zweiten Anbringabschnitt (110, 120) verbindet.

3. Chirurgische Verbindungsvorrichtung (100) nach einem der vorhergehenden Ansprüche, bei welcher die Anbringabschnitte (110, 120) ein Vorderende (111, 121) und ein Hinterende (112, 122) aufweisen, wobei der Beabstandungsabschnitt (130) mit dem jeweiligen Hinterende (112, 122) des ersten und zweiten Anbringabschnitts (110, 120) verbunden ist, wobei das Vorderende (111, 121) auf der dem Hinterende (112, 122) gegenüberliegenden Seite des jeweiligen Anbringabschnittes (110, 120) liegt.

4. Chirurgische Verbindungsvorrichtung (100) nach einem der vorhergehenden Ansprüche bei welchem die Anbringabschnitte (110, 120) bewegliche Teile (113, 114, 124) und Kontaktteile (115, 125) aufweisen, wobei die beweglichen Teile (113, 114, 124) relativ zu den Kontaktteilen (115, 125) elastisch beweglich sind.

5. Chirurgische Verbindungsvorrichtung (100) nach Anspruch 4, bei welcher die beweglichen Teile (113, 114, 124) elastisch quer zu einer das Vorderende (111, 121) und Hinterende (112, 122) verbindenden Ebene beweglich sind.

6. Chirurgische Verbindungsvorrichtung (100) nach Anspruch 4 oder 5, bei welcher die Kontaktteile (112, 115, 122, 125) flächig ausgebildet sind und/oder in einer Ebene liegen.

7. Chirurgische Verbindungsvorrichtung (100) nach einem der vorhergehenden Ansprüche, bei welcher die beweglichen Teile (113, 114, 124) in Richtung des Vorderendes (111, 121) abgeschrägt sind.

8. Chirurgische Verbindungsvorrichtung (100) nach einem der vorhergehenden Ansprüche, bei welcher Anschlagabschnitte (116, 126) jeweils zwischen dem Beabstandungsabschnitt (130) und den Anbringabschnitten (110, 120) vorgesehen sind, die als Anschlag beim Einführen der Anbringabschnitte (110, 120) in Schneidschlitze der Schneidblöcke (200, 300) dienen.

9. Chirurgische Verbindungsvorrichtung (100) nach einem der vorhergehenden Ansprüche, die eine Markervorrichtung (400) aufweist.

10. Chirurgische Verbindungsvorrichtung (100) nach einem der vorhergehenden Ansprüche mit einem am Beabstandungsahschnitt (130) angeordneten Haltegriff (136) und/oder wobei der Beabstandungsabschnitt einen Verstellmechanismus aufweist, mit welchem der Abstand zwischen dem ersten und zweiten Anbringabschnitt verstellbar ist.

11. Chirurgische Verbindungsvorrichtung (100) nach einem der vorhergehenden Ansprüche mit einer Patella-Schneidvorrichtung (800).

12. Chirurgisches System mit zwei Schneidblöcken (200, 300), mit der chirurgischen Verbindungsvorrichtung (100) nach einem der vorhergehenden Ansprüche und mit einem Einstellmechanismus (400).

13. Navigationssystem mit einer Navigationsvorrichtung (600) und dem chirurgischen System nach Anspruch 12.

14. Verwendung der Vorrichtung (100) nach einem der Ansprüche 1 bis 11 oder des Systems nach Anspruch 12 oder 13, um die zwei Schneidblöcke (200, 300) an einer ersten (20) und zweiten (10) Körperstruktur, die über ein Gelenk verbundenen sind, anzubringen, mit folgenden Schritten:
in einem Zustand, in dem der Einstellmechanismus (400) an der ersten Körperstruktur (20) befestigt ist und mit dem ersten Schneidblock (300) verbunden ist und die Verbindungsvorrichtung (100) den ersten (300) und zweiten (200) Schneidblock verbindet, wird mittels des Einstellmechanismus (400) die Lage des zweiten Schneidblocks (200) relativ zu dem ersten Körperteil (20) eingestellt,
der zweite Schneidblock (200) wird an der zweiten Körperstruktur (10) fixiert,
die Verbindungsvorrichtung (100) wird entfernt, so dass der mit dem Einstellmechanismus (400) verbundene zweite Schneidblock (200) relativ zum ersten Schneidblock (300) beweglich ist,
mittels des Einstellmechanismus (400) wird die Lage des ersten Schneidblocks (300) relativ zur ersten Körperstruktur (20) eingestellt,
der erste Schneidblock (300) wird an der ersten Körperstruktur (20) fixiert.

15. Verwendung nach Anspruch 14, bei welchem die Einstellung der Lage des ersten (300) und zweiten (200) Schneidblocks mit Hilfe der Detektion mindestens einer Markervorrichtung (500, 700) erfolgt, die zumindest zur Zeit der Einstellung der Lage des ersten Schneidblocks (300) ortsfest mit dem ersten Schneidblock (300) verbunden ist und zur Zeit der Einstellung der Lage des zweiten Schneidblocks (200) ortsfest mit dem zweiten Schneidblock (200) verbunden ist.
